# EUROPÄISCHE PATENTSCHRIFT

(11) **EP 0 895 491 B1**
(45) Veröffentlichungstag und Bekanntmachung des Hinweises auf die Patenterteilung: **29.01.2003**
(21) Anmeldenummer: 97919386.9
(22) Anmeldetag: 18.04.1997
(51) Int. Cl.: B25J 18/06, B25J 7/00, B25J 9/14

(54) **VERFAHREN ZUR PLANAREN HERSTELLUNG VON PNEUMATISCHEN UND FLUIDISCHEN MINIATURMANIPULATOREN**
METHOD FOR THE PRODUCTION OF FLAT PNEUMATIC AND FLUID MICROMANIPULATORS
PROCEDE DE FABRICATION DE MICROMANIPULATEURS PNEUMATIQUES ET HYDRAULIQUES PLATS

(30) Priorität: 23.04.1996 DE 19617852
(43) Veröffentlichungstag der Anmeldung: 10.02.1999
(73) Patentinhaber: Forschungszentrum Karlsruhe GmbH, 76133 Karlsruhe (DE)
(72) Erfinder: SCHULZ, Stefan, D-18109 Rostock (DE)
(74) Vertreter: Rückert, Friedrich, Dr.
(86) Internationale Anmeldenummer: EP9701954
(87) Internationale Veröffentlichungsnummer: WO97039861

(56) Entgegenhaltungen:
- WO-A-90/15697
- DE-A- 19 500 368
- GB-A- 2 240 083

## Beschreibung

Die Erfindung betrifft einen pneumatisch oder fluidisch betriebenen Miniaturmanipulator. Gattungsgemäße Manipulatoren sind aus der WO-A-90 156 97 bekannt.

Die Erfindung kann zur Herstellung von Manipulatoren genutzt werden, die z. B. zum Erreichen von schwer zugänglichen Gebieten angewandt und in der Mechanik, Kybernetik, Fluidtechnik, Pneumatik, Bewegungsphysik, Medizintechnik verwendet werden, und beispielsweise als Minimanipulator in der minimal invasiven Chirurgie und Neurochirurgie einsetzbar sind.

Die Verwendung von Flüssigkeiten oder Gasen als Antriebsmedium für Aktoren hat den Vorteil, dass große Energiemengen extern erzeugt und verlustfrei über feinste Übertragungsstrecken zum Wirkungsort transportiert werden können, um dort Arbeit zu verrichten.

Manipulatoren mit diesen Antriebsarten werden aus dreidimensionalen Komponenten mit einer dreidimensional arbeitenden Fertigungstechnologie hergestellt. Die technologische Beherrschbarkeit von z. B. schlauchförmigen Manipulatoren für die Neurochirurgie, beschränkt sich auf Durchmesser von ca. 2 - 5 mm, wobei mit bis zu drei Freiheitsgraden nur ein eingeschränkter Bewegungskreis zur Verfügung steht.

[R. Trapp, B. Neisius, HIT, E. Holler, IAI, M. Englert, Ing.-Büro für technische Kybenetik: Telemanipulator für die minimal invasive Chirurgie, Nachrichten, Forschungszentrum Karlsruhe GmbH, Jahrgang 27, 2-3/95, S. 145 - 154. A. Guber, W. Menz, IMT: MINOP - Entwicklung eines miniaturisierten neuroendoskopischen Operationssystems, Nachrichten, Forschungszentrum Karlsruhe GmbH, Jahrgang 27, 2-3/95, S. 155 - 160.]

In der WO 90/15697 wird eine Einrichtung für einen Drehpunkt verbindungsbildenden Gegenstand beschrieben. Ein solcher Druckmedium betriebener Gegenstand, z.B. für einen Roboterarm, ein Greif- oder Halteteil oder Ähnlichem, ist wieder ausrichtbar aus einer zusammengeklappten Stellung in eine gestreckte und umgekehrt. Der Gegenstand ist aus einem biegsamen Wandmaterial hergestellt, und zwar aus einem ersteren Wandtyp, das ein oder mehrere örtlich bestimmte Taschenformationen mit einer angeschlossenen Zuleitung für das Druckmedium hat und einen zweiten Wandtyp ohne solche Taschenformationen. Der erste Wandtyp ist angeschlossen, um durch Aufpumpen der Taschenformation deformiert zu werden und um die Breite und Länge des anliegenden Wandteils zusammen zu ziehen. Der zweite Wandtyp formt ein klammerndes Teil für die Wandteile des ersten Wandtyps. Der erste Wandteiltyp ist zum zweiten Wandteiltyp auf Abstand angeordnet, so dass der erste relativ zum zweiten durch Befüllen der Taschenformation/en zusammen geklappt und durch Abpumpen gestreckt werden kann.

Nachteile des derzeitigen Stands der Technik sind:

Einerseits sind der feinwerktechnischen Herstellung von pneumatisch- bzw. flüssigkeitsangetriebenen Manipulatoren Grenzen gesetzt in Bezug auf Miniaturisierung, Komplexität und Materialbelastung. Für viele Anwendungsgebiete wie z. B. in der Neurochirurgie, sind die herstellbaren Manipulatoren noch zu groß und zu unbeweglich.
Der technologische Aufwand steigt mit kleiner werdenden Strukturen, da entsprechend präzisere Werkzeuge für die Bearbeitung benötigt werden. Besonders die maschinelle Montage mehrerer dreidimensionaler Einzelkomponeten setzt sehr genaue Automaten mit vielen Freiheitsgraden voraus. Dadurch ist die automatische Produktion eingeschränkt und eine Massenproduktion behindert. Der technologische Aufwand steigt auch mit zunehmender Komplexität der hergestellten Manipulatoren, z. B. durch die steigende Anzahl der Freiheitsgrade, da eine größere Anzahl von Einzelkomponenten montiert werden muss.

Andrerseits ist mit der Einrichtung aus beweglichen Wänden, wie in der WO 90/15697 beschrieben, nur zwischen zwei Grenzgeometrien nämlich einem zusammengeklappten und gestreckten Zustand wechselbar, wenn auch Kräfte übertragen werden sollen.

Der Erfindung liegt die Aufgabe zugrunde, einen Miniaturmanipulator bereitzustellen, der pneumatisch oder fluidisch betrieben wird, mit dem ein Miniaturaktor durch ein gasförmiges oder flüssiges Medium angetrieben wird und mit dem beispielsweise wurmähnliche Manipulatoren mit vielen Aktoren herstellbar sind, deren Durchmesser nur einige Millimeter und kleiner als ein Millimeter betragen.

Erfindungsgemäß wird dieses Problem durch die Merkmale des Patenanspruchs 1, insbesondere durch die kennzeichnenden Merkmale gelöst.

In den Unteransprüchen 2 und 3 sind Eigenschaften des Miniaturmanipulators beschrieben, die eine vorteilhafte Handhabung zulassen.

Mit der Erfindung werden folgende Vorteile erzielt:
1. daß sich mit dem beschriebenen Aufbau sehr kleine Manipulatoren herstellen lassen, die über viele Freiheitsgrade verfügen;
2. daß eine Montage kleinster Einzelkomponenten nicht möglich ist;
3. daß ohne erheblichen technologischen Mehraufwand eine zunehmende Anzahl von Freiheitsgraden und eine Umstellung auf eine neue Form realisierbar ist;
4. daß Form und Funktion des Manipulators in einem großem Rahmen frei gestaltbar sind;
5. daß bei steigender Komplexität des Bewegungsapparates der technologische Aufwand unverändert bleibt;
6. daß mit jedem technologischen Schritt mehrere Manipulatoren gleichzeitig bearbeitet werden können;
7. daß die Massenherstellung die Realisierung eines Einmalproduktes mit seinen Vorteilen, z. B. keine Wartung und Reinigung, ermöglicht;
8. daß für die zweidimensionale Konstruktion und Bearbeitung eine Vielzahl von rechnergestützten Gestaltungshilfen zur Verfügung stehen;
9. daß der Manipulator aus preiswertem Folienmaterial herstellt wird;
10.daß der Manipulator vollständig aus Kunststoff gefertigt werden kann und somit keine Beeinträchtigung für durchstrahlende Aufnahmen darstellt;
11.daß sich der Manipulator durch seine flache Bauform und seine flexible Eigenschaft im drucklosen Zustand durch kleine Öffnungen in Hohlräumen bringen, sowie platzsparend transportieren und lagern läßt.

Die Erfindung wird nachstehend an mehreren Ausführungsbeispielen näher erläutert. Es zeigen:
Fig. 1 den Aufbau allgemein,
Fig. 2 ein Ausführungsbeispiel des Aufbaus,
Fig. 3 eine schematische Darstellung der Heizung,
Fig. 4 Darstellung eines Miniaturaktors mit einem Bewegungsprinzip, das auf Kontraktion beruht,
Fig. 5 Darstellung eine Miniaturaktors mit einem Bewegungsprinzip, das auf Expansion beruht.

Die Wirkungsweise wird, wie folgt, beschrieben:

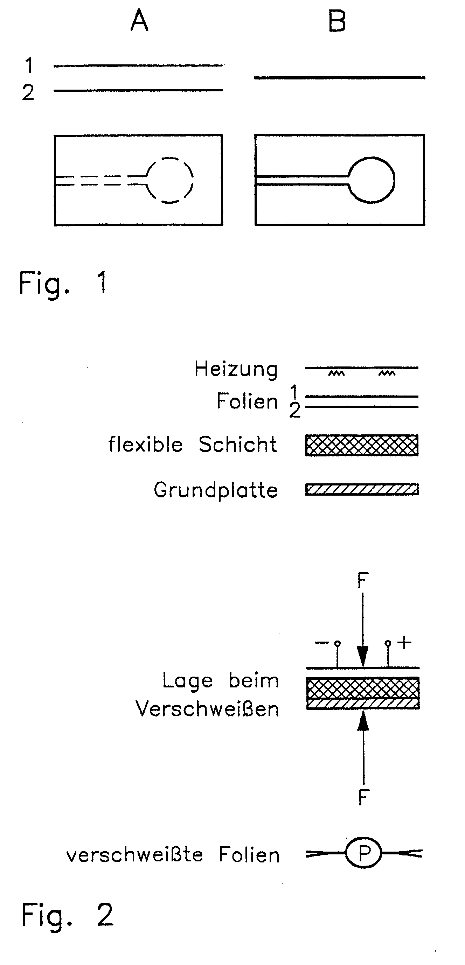

Nach Figur 1 ist der Aufbau für die planare Herstellung von pneumatischen und fluidischen Miniaturmanipulatoren, entsprechend Patentanspruch 1, dadurch gekennzeichnet, daß mindestens zwei oder mehr Folien übereinander geschichtet sind und miteinander zu einem Folienpaar verbunden werden, so daß Kammern und Kanäle entstehen, die in ihrer Gestalt beliebig sind.

Fig. 1/Abb. A zeigt, wie Folie 1 und Folie 2 übereinander geschichtet sind und im Anschluß entlang der gestrichelten Linie miteinander verbunden werden.

Die Verbindung aus Folie 1 und Folie 2 wird in Fig. 1/Abb. B als Folienpaar 1 dargestellt. Dieses besitzt nun eine, über einen Kanal aufpumpbare Kammer.

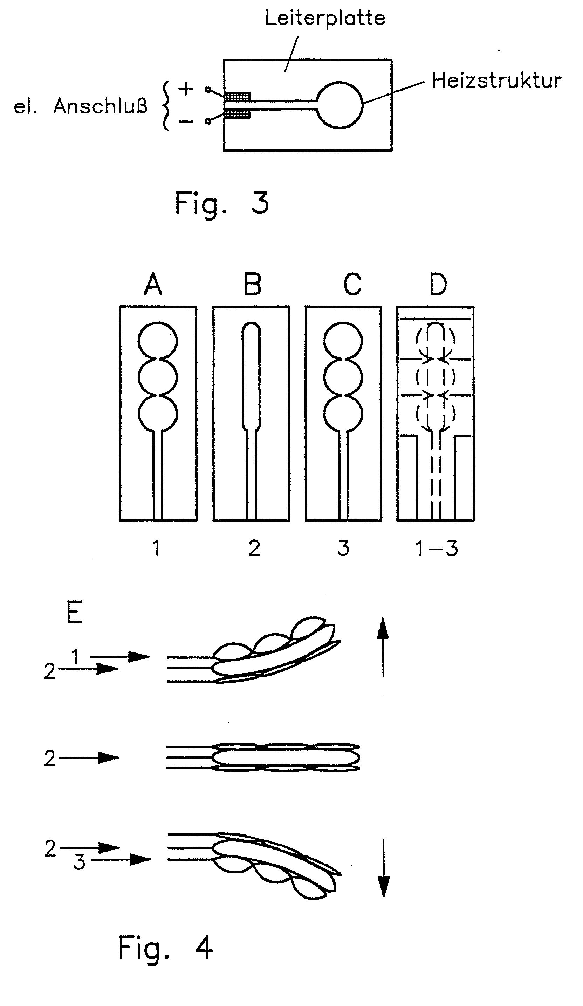

Nach Figur 2 erfolgt die Verbindung zweier Folien entlang einer in Fig. 1/Abb. A gestrichelt gezeichneten Linie durch Verschweißen. Zwei Folien werden zwischen Heizung und Grundplatte gepreßt. Die flexible Schicht stellt einen guten Kontakt her zwischen den Folien und der Heizung. Die Heizung besitzt, vergleichbar einem Stempel, die gewünschte Form der zu erzeugenden Struktur z.B. Kanäle und Kammern. Folie 1 und Folie 2 verschmelzen entlang der Heizstruktur miteinander. Eine mögliche Ausführung der Heizung ist in Fig. 3 dargestellt.

Nach Figur 3 kann die unter Figur 2 beschriebene Heizung durch eine Leiterplattenstruktur realisiert werden.

Die Struktur des Leiterzuges wird durch die zu realisierenden Folienverbindung bestimmt. Wird der Leiterzug vom Strom durchflossen, heizt sich der Leiterzug auf. Diese Erwärmung führt zum Verschmelzen der Folien entlang des Leiterzuges und damit zum festen Verbund der Folien an der gewünschten Stelle.

Nach Figur 4 werden drei, hergestellte Folienpaare zu einem Manipulator verbunden. Die Verbindung erfolgt entlang der in Fig. 4/Abb. D gezeichneten breiten Linien. Das Folienpaar 2 stellt dabei entsprechend Patentanspruch 7 und 8 ein Stützelement dar.

Durch Aufpumpen und Entleeren der Folienelemente entsprechend Fig. 4/Abb. E wird eine Bewegung des Manipulators dadurch erzielt, dass die mit der Verformung der Folien verbundene Kontraktion der Kammern für eine zusammenführend Bewegung genutzt wird.

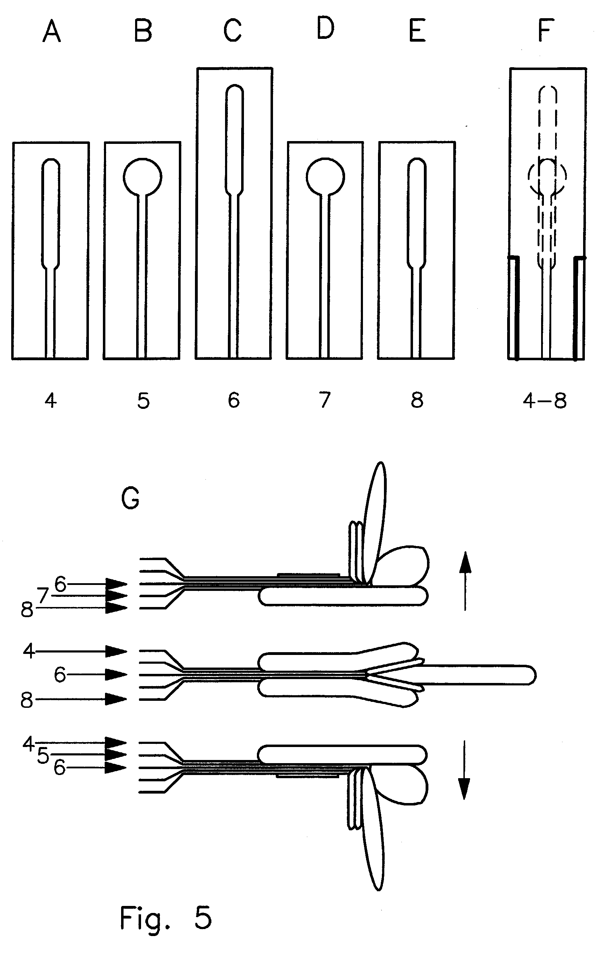

Nach Figur 5 werden fünf, hergestellte Folienpaare zu einem Manipulator verbunden. Die Verbindung erfolgt entlang der in Fig. 5/Abb. F gezeichneten breiten Linien. Die Folienpaare 4, 6 und 8 stellen, Stützelemente dar.

Durch Aufpumpen und Entleeren der Folienpaare entsprechend Fig. 5/Abb. G wird eine Bewegung des Manipulators dadurch erzielt, dass die mit der Verfor mung der Folien verbundene Volumenzunahme für die Auseinanderbewegung zweier Punkte genutzt wird.

## Patentansprüche

1. Miniaturmanipulator,
der pneumatisch oder fluidisch betrieben wird,
der aus druckmediumbetriebenen, mit Zuleitung versehenen und damit auf- und abpumpbaren Kammern aus biegsamem Wandmaterial, wobei das Druckmedium gasförmig oder flüssig ist, aufgebaut ist,
**dadurch gekennzeichnet,**
**dass** mindestens vier Folien aus dem Wandmaterial zusammen-bzw. aufeinandergelegt sind und zwei unmittelbar benachbarte Folien entlang mindestens einer Linie miteinander verbunden bzw. verschweißt sind,
wobei:
zwei unmittelbar benachbarte
Folien jeweils eine Struktur aus mindestens einer Kammer und einem Kanal zum Auf- und Abpumpen des Druckmediums bilden,
die strukturbildenden Folienpaare miteinander über mindestens eine Linie zu dem Mikromanipulator verbunden sind,
durch Aufpumpen mindestens einer Struktur eine vorgesehen ausgerichtete, dreidimensionale Gestalt eingestellt und kraftübertragungsfähig gehalten werden kann.

2. Miniaturmanipulator nach Anspruch 1,
**dadurch gekennzeichnet,**
**dass** die strukturbildenden Folienpaare unabhängig voneinander auf- und abpumpbar sind.

3. Miniaturmanipulator nach Anspruch 2,
**dadurch gekennzeichnet,**
**daß** eines der Folienpaare im aufgepumpten Zustand als Stützstruktur dient, um die anliegenden auszulenken und entsprechend ihrer Befüllung auszurichten.

## Claims

1. Miniature manipulator
operated by pneumatic or fluidic means, made up of compartments out of a flexible wall material which are operated by a pressurization medium and equipped with a feed line allowing them to be inflated and deflated with a gaseous or liquid pressurization medium,
in which at least four foils of the wall material are folded or put on top of each other, and two directly adjacent foils are joined and welded, respectively, along at least one line,
where two immediately adjacent foils each constitute a structure consisting of at least one compartment and one channel for supplying and removing the pressurization medium,
the foil pairs constituting the structures are interconnected by at least one line leading to the micromanipulator;
inflating at least one structure establishes a three-dimensional shape aligned as planned and held in a state capable of transmitting forces.

2. Miniature manipulator as claimed in Claim 1,
in which the foil pairs constituting the structure can be inflated and deflated independently.

3. Miniature manipulator as claimed in Claim 2,
in which one of the foil pairs in the inflated state acts as a support structure deflecting the contacting foil pairs and aligning them in accordance with their level of inflation.

## Revendications

1. Manipulateur miniature,
commandé par voie pneumatique ou fluidique,
constitué de chambres conduites par un fluide comprimé, munies de lignes d'admission et par conséquent gonflables et évacuables, aux parois en matériau souple, le fluide comprimé étant un milieu gazeux ou liquide,
**caractérisé en ce que**
quatre feuilles au minimum du matériau de paroi sont assemblées ou empilées respectivement et que deux feuilles directement voisines sont raccordées ou soudées entre elles le long d'une ligne au minimum,
deux feuilles directement voisines formant une structure composée au moins d'une chambre et d'un canal pour injecter et évacuer le fluide comprimé,
les paires de feuilles formant une structure sont raccordées entre elles par au moins une ligne conduisant au manipulateur miniature,
le gonflage d'une structure au moins permet de réaliser une forme d'orientation prédéfinie, tridimensionnelle pouvant être maintenue capable de transmettre une force motrice.

2. Manipulateur miniature selon la revendication 1,
**caractérisé en ce que**
les paires de feuilles formant une structure peuvent être gonflées et évacuées indépendamment les unes des autres.

3. Manipulateur miniature selon la revendication 2,
**caractérisé en ce que**
une paire de feuilles sert de structure de support à l'état gonflé pour diriger les paires adjacentes et les orienter en fonction du fluide injecté.
